# EUROPÄISCHE PATENTSCHRIFT

(11) **EP 0 664 109 B1**
(45) Veröffentlichungstag und Bekanntmachung des Hinweises auf die Patenterteilung: **21.08.1996**
(21) Anmeldenummer: 94120022.2
(22) Anmeldetag: 16.12.1994
(51) Int. Cl.: A61F 5/01

(54) **Sprunggelenkorthese**
Ankle joint orthosis
Orthèse pour la cheville

(30) Priorität: 24.12.1993 DE 9319990 U
(43) Veröffentlichungstag der Anmeldung: 26.07.1995
(73) Patentinhaber: Medi Bayreuth GmbH & Co. KG, D-95448 Bayreuth (DE); PAROMED Medizintechnik GmbH, D-83115 Markt Neubeuern (DE)
(72) Erfinder: Reithofer, Alfred, D-83026 Rosenheim (DE)
(74) Vertreter: Merten, Fritz

(56) Entgegenhaltungen:
- EP-A- 0 297 157
- WO-A-88/09156
- DE-A- 3 803 304
- DE-U- 8 700 201
- DE-U- 9 319 990
- US-A- 2 800 900
- US-A- 5 113 877

## Beschreibung

Die Erfindung betrifft eine Sprunggelenkorthese mit den Merkmalen des Oberbegriffs des Anspruchs 1.

Eine Sprunggelenkorthese zur Stabilisierung des inneren und äußeren Sprunggelenks, bestehend aus einer medialen und einer lateralen Schale aus einem Kunststoff, wobei sich die Schalen von dem Fersenbereich über den Malleolus hinweg bis in den unteren Wadenbereich erstrecken, wobei die Schalen innen über die gesamte Auflagefläche mit einem nachgiebigen Polster ausgekleidet sind und wobei Klettverschlüsse zum Befestigen und Fixieren der Schalen vorhanden sind, ist aus der DE-C-29 13 606 bekannt. Die WO 90/01911 zeigt eine orthopädische Vorrichtung mit Gel-Kissen, welche schalenartig um Gliedmaßen angebracht wird, und die gekühlt oder aufgeheizt werden kann. Die DE-U-89 07 464 zeigt eine steigbügelartige, einteilige und mit Ausnehmungen für die Ferse und die malleoli versehene Sprunggelenkorthese.

Aufgabe der vorliegenden Erfindung ist es, eine Sprunggelenkorthese zu schaffen, die eine hohe Stabilisierung des Sprunggelenks ermöglicht, die den Thalus-Vorschub wirksam vermindert, die sich beschwerdefrei tragen und sich gut um das Sprunggelenk befestigen läßt.

Diese Aufgabe wird mit den im kennzeichnenden Teil des Anspruchs 1 genannten Merkmalen gelöst. Fortbildungen und vorteilhafte Ausführungen der Erfindung sind in den weiteren Ansprüchen umfaßt.

Erfindungsgemäß ist bei einer Sprunggelenkorthese der eingangs geschilderten Art eine Schale, vorzugsweise die laterale Schale, L-förmig ausgeführt und greift unter den Fuß zwischen Ferse und Ballen. Beide Schalen sind mittels eines Klettbandes unter dem Fuß verbindbar, wobei ein an einer der beiden Schalen befestigtes Klettband unter dem Fuß hindurchgeführt und an der anderen Schale an einem Klettverschluß fixierbar ist. Beide Schalen weisen im Bereich der empfindlichen Malleolen Durchbrüche auf. Eine optimale Druckverteilung über die gesamte Auflagefläche wird durch die Polster an den Innenseiten der Schalen erreicht. Die L-Form der einen Schale ermöglicht eine verbesserte Fußfassung.

Vorteilhafterweise erstreckt sich das Polster, das ein Silikonpolster, vorzugsweise eine mit flüssigem oder gelartigem Silikon gefüllte Hülle sein kann, über die Durchbrüche hinweg.

Im oberen Bereich sind die Schalen mittels eines ringförmig um den Unterschenkel verlaufenden Klettbandes am Unterschenkel fixierbar. Eine optimale Befestigung um das Sprunggelenk wird dadurch erzielt, daß die Schalen im mittleren Bereich mit vorne und hinten von medial unterhalb des malleolus medialis nach lateral bis über den malleolus lateralis verlaufenden und dort umgelenkten und oberhalb des malleolus medialis an der medialen Schale fixierbaren Klettbändern um das Sprunggelenk und den unteren Unterschenkel befestigbar sind.

Die Umlenkung erfolgt in einfacher Weise durch Schlitze in der lateralen Schale.

Die beschriebene Sprunggelenkorthese eignet sich zur postoperativen, konventionellen, als auch präventiven Behandlung von Sprunggelenkverletzungen.

Im folgenden wird die Erfindung anhand von Zeichnungen beispielhaft näher beschrieben. Dabei zeigen:
- Fig. 1: die mediale Ansicht einer Sprunggelenkorthese;
- Fig. 2: die laterale Ansicht einer Sprunggelenkorthese;
- Fig. 3: eine Abwandlung der in Fig. 1 und 2 gezeigten Sprunggelenkorthese in medialer Ansicht;
- Fig. 4: eine Abwandlung der in Fig. 1 und 2 gezeigten Sprunggelenkorthese in lateraler Ansicht.

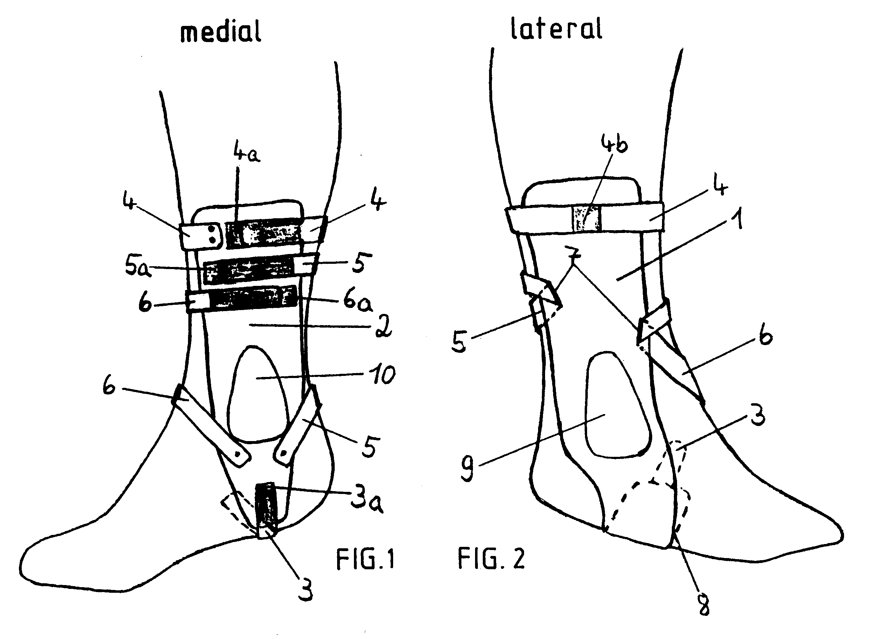

Wie in Fig. 1 und Fig. 2 dargestellt, besteht die gezeigte Sprunggelenkorthese aus einer medialen Schale 2 und einer lateralen Schale 1 aus einem Kunststoff. Die Schalen 1, 2 erstrecken sich von dem Fersenbereich über den Malleolus hinweg bis in den unteren Wadenbereich. Die laterale Schale 1 ist L-förmig ausgeführt ist und greift mit einem Ansatz 8 unter den Fuß zwischen Ferse und Ballen. Beide Schalen 1, 2 sind mittels eines Klettbandes 3 unter dem Fuß verbindbar, wobei ein an dem Ansatz 8 der lateralen Schale 1 befestigtes Klettband 3 unter dem Fuß hindurchgeführt und an der medialen Schale 2 an einem Klettverschluß 3a fixierbar ist. Beide Schalen 1, 2 weisen im Bereich der Malleolen Durchbrüche 9, 10 auf.

In ihrem oberen Bereich sind die Schalen 1, 2 mittels eines endseitig an der medialen Schale befestigten und ringförmig um den Unterschenkel verlaufenden Klettbandes 4 mit Klettverschlüssen 4a, 4b an beiden Schalen 1, 2 am Unterschenkel fixierbar. Im mittleren Bereich sind die Schalen 1, 2 mit vorne und hinten von medial unterhalb des malleolus medialis nach lateral bis über den malleolus lateralis verlaufenden und dort umgelenkten und oberhalb des malleolus medialis mit Klettverschlüssen 5a, 6a an der medialen Schale 2 fixierbaren Klettbändern 5, 6 um das Sprunggelenk und den unteren Unterschenkel befestigbar. Die Umlenkung erfolgt durch Schlitze 7 in der lateralen Schale 1.

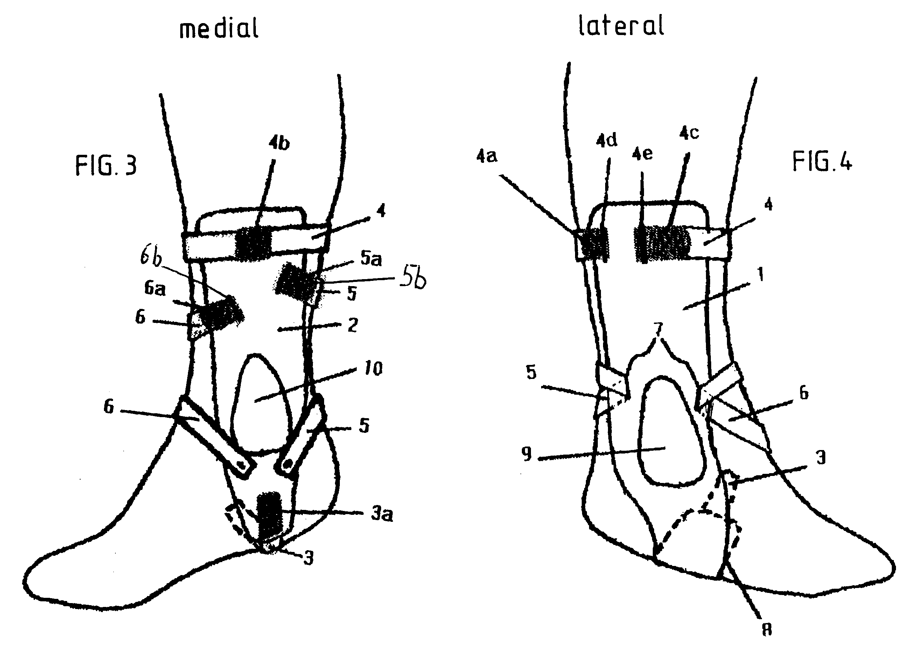

Die in den Fig. 3 und 4 gezeigte Abwandlung bezieht sich auf die Befestigungen der Klettbänder 4, 5 und 6. Die Sprunggelenkorthese besteht auch hier aus einer medialen Schale 2 und einer lateralen Schale 1 aus einem Kunststoff. Die Schalen 1, 2 erstrecken sich von dem Fersenbereich über den Malleolus hinweg bis in den unteren Wadenbereich. Die laterale Schale 1 ist L-förmig ausgeführt und greift mit einem Ansatz 8 unter den Fuß zwischen Ferse und Ballen. Beide Schalen 1, 2 sind mittels eines Klettbandes 3 unter dem Fuß verbindbar, wobei ein an dem Ansatz 8 der lateralen Schale 1 befestigtes Klettband 3 unter dem Fuß hindurchgeführt und an der medialen Schale 2 an einem Klettverschluß 3a fixierbar ist. Beide Schalen 1, 2 weisen im Bereich der Malleolen Durchbrüche 9, 10 auf.

In ihrem oberen Bereich sind die Schalen 1, 2 mittels eines ringförmig um den Unterschenkel verlaufenden Klettbandes 4 mit Klettverschlüssen 4a, 4b und 4c an beiden Schalen 1, 2 am Unterschenkel fixierbar. Dabei werden die Enden des Klettbandes 4 um Umlenkstege 4d, 4e, die auch mit Umlenkrollen ausgestattet sein können, umgelenkt und an sich selbst fixiert. Im mittleren Bereich sind die Schalen 1, 2 mit vorne und hinten von medial unterhalb des malleolus medialis nach lateral bis über den malleolus lateralis verlaufenden und dort umgelenkten und oberhalb des malleolus medialis mit Klettverschlüssen 5a, 6a an der medialen Schale 2 fixierbaren Klettbändern 5, 6 um das Sprunggelenk und den unteren Unterschenkel befestigbar. Die Umlenkung erfolgt durch Schlitze 7 in der lateralen Schale 1 und die Befestigung der Klettverschlüsse 5a, 6a erfolgt durch endseitiges Umlenken über Umlenkstege 5b, 6b, die auch mit Umlenkrollen versehen sein können.

## Patentansprüche

1. Sprunggelenkorthese zur Stabilisierung des inneren und äußeren Sprunggelenks,
bestehend aus einer medialen (2) und einer lateralen Schale (1) aus einem Kunststoff,
wobei sich die Schalen von dem Fersenbereich über den malleolus hinweg bis in den unteren Wadenbereich erstrecken,
wobei die Schalen (1, 2) innen über die gesamte Auflagefläche mit einem nachgiebigen Polster ausgekleidet sind,
und wobei Klettverschlüsse (4a, 4b) zum Befestigen und Fixieren der Schalen (1, 2) vorhanden sind,
**dadurch gekennzeichnet,**
daß eine Schale, vorzugsweise die laterale Schale (1), L-förmig ausgeführt ist und unter den Fuß zwischen Ferse und Ballen greift,
daß beide Schalen (1, 2) mittels eines Klettbandes (3) unter dem Fuß verbindbar sind, wobei ein an einer der beiden Schalen befestigtes Klettband (3) unter dem Fuß hindurchgeführt und an der anderen Schale an einem Klettverschluß (3a) fixierbar ist,
und daß beide Schalen (1, 2) im Bereich der Malleolen Durchbrüche (9, 10) aufweisen.

2. Sprunggelenkorthese nach Anspruch 1,
**dadurch gekennzeichnet**,
daß sich das Polster über die Durchbrüche (9, 10) hinweg erstreckt.

3. Sprunggelenkorthese nach Anspruch 1,
**dadurch gekennzeichnet**,
daß das Polster ein Silikonpolster ist.

4. Sprunggelenkorthese nach Anspruch 3,
**dadurch gekennzeichnet**,
daß das Polster eine mit einem flüssigen oder gelartigen Silikon gefüllte Hülle ist.

5. Sprunggelenkorthese nach Anspruch 1,
**dadurch gekennzeichnet**,
daß die Schalen (1, 2) im oberen Bereich mittels eines ringförmig um den Unterschenkel verlaufenden Klettbandes (4) am Unterschenkel fixierbar sind.

6. Sprunggelenkorthese nach Anspruch 5,
**dadurch gekennzeichnet**,
daß die Schalen (1, 2) im mittleren Bereich mit vorne und hinten von medial unterhalb des malleolus medialis nach lateral bis über den malleolus lateralis verlaufenden und dort umgelenkten und oberhalb des malleolus medialis an der medialen Schale (2) fixierbaren Klettbändern (5, 6) um das Sprunggelenk und den unteren Unterschenkel befestigbar sind.

7. Sprunggelenkorthese nach Anspruch 6,
**dadurch gekennzeichnet**,
daß die Umlenkung durch Schlitze (7) in der lateralen Schale (1) erfolgt.

## Claims

1. An ankle joint orthosis for stabilizing the inner and outer ankle joint, comprising a medial (2) and a lateral shell (1) made of a plastics material, wherein the shells extend from the heel region beyond the malleolus to the lower calf region, wherein the shells (1,2) are lined internally over the whole contact surface with a soft pad, and wherein there are fastenings (4a,4b) for securing and fixing the shells (1,2) in position, characterized in that one shell, preferably the lateral shell (1), is L-shape in design and reaches underneath the foot between the heel and the ball of the foot, in that the two shells (1,2) can be joined underneath the foot by means of a fastening strap (3), a fastening strap (3) secured to one of the two shells being passed underneath the foot and being fixable to the other shell by a fastening (3a), and in that both shells (1,2) have openings (9,10) in the region of the malleoli.

2. An ankle joint orthosis according to Claim 1, characterized in that the pad extends beyond the openings (9,10).

3. An ankle joint orthosis according to Claim 1, characterized in that the pad is a silicone pad.

4. An ankle joint orthosis according to Claim 3, characterized in that the pad is an envelope filled with a liquid or gel-like silicone.

5. An ankle joint orthosis according to Claim 1, characterized in that the shells (1,2) are fixable in the upper region to the lower leg by means of a fastening strap (4) extending circularly around the lower leg.

6. An ankle joint orthosis according to Claim 5, characterized in that the shells (1,2) may be secured in the central region around the ankle joint and the lower part of the lower leg by means of front and rear fastening straps (5,6) which extend from the medial side below the malleolus medialis to the lateral side above the malleolus lateralis, and are there turned back, and which may be fixed above the malleolus medialis to the medial shell (2).

7. An ankle joint orthosis according to Claim 6, characterized in that the deflection is effected by slits (7) in the lateral shell (1).

## Revendications

1. Orthèse tarsienne, destinée à stabiliser l'articulation astragalo-calcanéo-scaphoïdienne et l'articulation tibio-tarsienne, constituée d'une enveloppe médiane (2) et d'une enveloppe latérale (1) en une matière plastique, les gaines de la zone du talon s'étendant, en passant sur la malléole, jusqu'à la partie inférieure du mollet ; les gaines (1, 2) étant, sur leur face intérieure, sur la totalité de leur surface d'appui, revêtues d'un bourrelet élastique ; des fermetures de type à accrochage à crampons (4a, 4b) étant présentes pour assujettir et fixer les gaines (1, 2), caractérisée en ce qu'une gaine, de préférence la gaine latérale (1), est réalisée en forme de L et passe sous le pied entre le talon et l'éminence du gros orteil ; que les deux gaines (1, 2) peuvent être reliées l'une à l'autre par une bande de type à accrochage à crampons (3) en-dessous du pied, une bande de type à accrochage à crampons (3), fixée à l'une des deux gaines, passant en-dessous du pied, et pouvant être assujettie à l'autre gaine, à l'aide d'une fermeture de type à accrochage à crampons (3a) ; et que les deux gaines (1, 2) présentent des ouvertures (9, 10) dans la zone des malléoles.

2. Orthèse tarsienne selon la revendication 1, caractérisée en ce que le bourrelet s'étend sur les ouvertures (9, 10).

3. Orthèse tarsienne selon la revendication 1, caractérisée en ce que le bourrelet est un bourrelet de silicone.

4. Orthèse tarsienne selon la revendication 3, caractérisée en ce que le bourrelet est une enveloppe remplie d'une silicone liquide ou de type gel.

5. Orthèse tarsienne selon la revendication 1, caractérisée en ce que les gaines (1, 2) peuvent être assujetties à la jambe à l'aide d'une bande de type à accrochage à crampons (4), courant d'une manière annulaire autour de la jambe.

6. Orthèse tarsienne selon la revendication 5, caractérisée en ce que les gaines (1, 2) peuvent être fixées, autour de l'articulation tarsienne et de la jambe, dans leur zone centrale, avec des bandes de type à accrochage à crampons (5, 6), courant à l'avant et à l'arrière, à partir d'une zone médiale située en-dessous de la malléole médiane, vers une position latérale, jusqu'à passer au-dessus de la malléole latérale, pour y changer de direction, et pouvant être fixées, au-dessus de la malléole médiale, à la gaine médiane (2).

7. Orthèse tarsienne selon la revendication 6, caractérisée en ce que le changement de direction est réalisé grâce à des fentes (7) aménagées dans la gaine latérale (1).
